Europäisches Patentamt

European Patent Office

Office européen des brevets

(19)

(11) **EP 1 472 521 B1**

(12) **EUROPÄISCHE PATENTSCHRIFT**

(45) Veröffentlichungstag und Bekanntmachung des Hinweises auf die Patenterteilung:
**04.10.2006  Patentblatt 2006/40**

(21) Anmeldenummer: **03702569.9**

(22) Anmeldetag: **30.01.2003**

(51) Int Cl.:
*G01N 21/49* *(2006.01)*          *G01N 21/85* *(2006.01)*
*G01N 33/18* *(2006.01)*

(86) Internationale Anmeldenummer:
**PCT/EP2003/000929**

(87) Internationale Veröffentlichungsnummer:
**WO 2003/067228 (14.08.2003 Gazette 2003/33)**

(54) **VERFAHREN FÜR UNTERSUCHUNGEN AN FLÜSSIGKEITEN SOWIE VORRICHTUNG HIERFÜR**

METHOD FOR ANALYSING LIQUIDS AND DEVICE THEREFOR

PROCEDE POUR L'ANALYSE DE LIQUIDES ET DISPOSITIF CORRESPONDANT

(84) Benannte Vertragsstaaten:
**AT BE BG CH CY CZ DE DK EE ES FI FR GB GR HU IE IT LI LU MC NL PT SE SI SK TR**

(30) Priorität: **06.02.2002  DE 10204963**

(43) Veröffentlichungstag der Anmeldung:
**03.11.2004  Patentblatt 2004/45**

(73) Patentinhaber: **ISCO, Inc.**
**Lincoln**
**Nebraska 68504 (US)**

(72) Erfinder:
• **SIEPMANN, Friedrich, W.**
**64823 Darmstadt (DE)**

• **GAHR, Achim**
**61377 Goldbach (DE)**

(74) Vertreter: **Hahn, Christian et al**
**Endress + Hauser**
**(Deutschland)AG +Co. KG**
**PatServe**
**Colmarer Strasse 6**
**79576 Weil am Rhein (DE)**

(56) Entgegenhaltungen:
**EP-A- 0 459 846          EP-A- 0 634 645**
**EP-A- 1 013 326          WO-A-97/21088**
**WO-A-99/43621          US-A- 4 194 391**
**US-A- 4 313 340**

EP 1 472 521 B1

**Beschreibung**

Die Erfindung betrifft ein Verfahren zur Bestimmung der Inhaltsstoffe eines flüssigen Mediums mit einer Lichtquelle und einem optischen Detektor, beispielsweise einem Spektrometer, mit mindestens einem Messstrahl und mindestens einem Referenzstrahl, wobei mindestens ein Messstrahl durch das zu untersuchende Medium geführt und mindestens ein Referenzstrahl außerhalb des zu untersuchenden Mediums geführt wird.

Die für solche Verfahren verwendeten, fotometrisch messenden Sonden für den "in situ"-Einsatz zur Bestimmung der Inhaltsstoffe eines Fluides, zum Beispiel Flusswasser oder Abwasser, haben üblicherweise eine Lichtquelle und einen optischen Detektor, beispielsweise ein Spektrometer, mit mindestens einem Messstrahl und mindestens einem Referenzstrahl, wobei das Licht der Lichtquelle ggf. aufgefächert und mittels mindestens einer optischen Linse zu einem im wesentlichen parallelen Strahl gebündelt wird.

Spektrometer mit Mess- und Referenzstrahlen sind aus DE 3 248 070 AI und DE 3 340 570 AI und zur "in situ"-Messung aus AT-A 2167/99 bekannt.

Die DE 3 248 070 AI betrifft einen Infrarot-Analysator mit einem Strahl der geteilt und einerseits durch eine Messküvette, andererseits durch eine Referenzküvette geführt wird.

Die DE 3 340 570 A1 betrifft ein Spektralfotometer, bei dem der Strahl ebenfalls in einen Messstrahl und einen Referenzstrahl geteilt wird, allerdings zeitversetzt durch einen Drehspiegel. Dabei ist ein gemeinsamer Detektor für beide Strahlen vorgesehen. Um sicherzustellen, dass beide Teilstrahlen die gleiche Wellenlänge aufweisen, wird die Frequenzverschiebung im Monochromator nur dann vorgenommen, wenn keine Messung erfolgt.

Beide Vorrichtungen sind diskret aufgebaut, d.h. aus mehreren Einheiten bestehend, die zwar ein gemeinsames Gehäuse aufweisen können, das es aber nicht erlaubt, die Apparatur als Gesamtes in das zu messende Fluid einzutauchen, sondern es notwendig macht, gezogene Proben in entsprechenden Behältern wie Küvetten od. dgl. in die Apparatur einzubringen.

Die AT-A 2167/99 betrifft eine Spektralsonde zur "in situ"-Messung. Bei dieser Sonde wird der Messstrahl durch ein lichtdurchlässiges Fenster in das zu untersuchende Fluid und durch ein weiteres lichtdurchlässiges Fenster wieder in die Sonde geführt. Der Referenzstrahl wird nur im Inneren der Sonde geführt, ohne die das Fluid berührenden Fenster zu passieren.

Es gibt auch optische Sonden, die in das Fluid getaucht werden und "in situ" messen. Diese arbeiten allerdings nicht spektrometrisch, sondern auf nur eine Wellenlänge oder auf das Integral eines Wellenbereichs beschränkt und messen die Trübung des Fluids oder die Konzentration eines einzelnen speziellen Inhaltsstoffes. Die letztgenannten Verfahren können die optische Qualität der Fenster nicht untersuchen, da die Referenzstrahlen, soweit vorhanden, im Inneren der Gehäuse verlaufen. Die optische Qualität der Fenster beeinflusst aber die Qualität der Messung ganz wesentlich, da vor allem bei der Messung von Abwasser Fensterverfärbungen und Bakterienbewuchs nicht zu verhindern sind und auch mit mechanischen Reinigungen wie z. B. Scheibenwischern die optische Qualität der Fenster nicht garantiert werden kann.

Weiter ist es den genannten Systemen nicht möglich, mechanisch absetzbare Inhaltsstoffe, wie z. B. Belebtschlamm im Belebungsbecken einer Kläranlage vom Abwasser zu separieren. So müssen den Systemen zum Messen von Belebtschlamm Absetzeinheiten vorgeschaltet werden und dadurch geht insbesondere der Wert einer "in situ"-Messung verloren.

Der Bedarf an "in situ"-Messungen von Wässern, insbesondere von Flusswasser, Abwasser und Prozesswässern in Rohrleitungen, steigt. Mit der Spektralfotometrie lassen sich Kenngrößen wie z. B. Nitrat und der SAK (Spektraler Absorptions-Koeffizient) direkt messen. In Kombination mit heute verfügbaren mathematischen Optimierungsverfahren, wie z. B. Neuronalen Netzen, lassen sich bei qualifizierter Messtechnik auch Summenparameter, wie z. B. der TOC und der CSB, als Ersatzparameter bereitstellen.

Aus US-A-4 313 340 ist das Merkmal bekannt, dass bei einer Vorrichtung zur Bestimmung der Absetzgeschwindigkeit von Feststoffen ein Kolben bei seiner Hubbewegung die durchsichtige Wand der Messkammer reinigt.

Aus US-A-4 194 391 ist es bei einem Gerät zur Bestimmung der Absetzgeschwindigkeit von Feststoffen bekannt, dass ein Kolben die zu untersuchende Flüssigkeit in den Messraum saugt. Weiterhin wird eine Referenzmessung durch eine in der Kolbenstange befindliche Bohrung durchgeführt.

Die Erfindung hat nun das Ziel, das Verfahren und die Vorrichtung so zu gestalten, dass Mess- und Referenzstrahl die gleichen optischen Gläser zu passieren haben, mit den gleichen Trübungen und Verschmutzungen, und dass ein Messraum entsteht, der es ermöglicht, absetzbare Stoffe aus dem Bereich des Mess- und Referenzstrahls zu entfernen.

Die Aufgabe wird erfindungsgemäß durch ein Verfahren gemäß Anspruch 1 gelöst. Ein längsbeweglicher Kolben oder Kolbenschieber saugt das zu untersuchende Medium in einen Messraum und entfernt es aus diesem, und der Kolben oder Kolbenschieber säubert bei seiner Hubbewegung Fenster im optischen Strahlengang. Das zu messende Medium wird mittels des Kolbens in einen Glaszylinder gesaugt. Die optische Achse, bestehend aus einer Lichtquelle, mindestens einer optischen Linse, die das Licht zu einem im wesentlichen parallelen Strahl bündelt, mindestens einer optischen Linse, die das Licht nach Verlassen des Messmediums auf den Eintrittspunkt eines Lichtleiters oder den Einlass eines Spektrometers oder Fotodetektors lenkt, ist quer zur Zylinderachse angeordnet. Die Zylinderachse weist z. B.

senkrecht nach oben. Die optische Achse und die Achse des Messzylinders schneiden sich z. B. unter einem Winkel von 90°.

Erfindungsgemäß wird mindestens ein Messstrahl durch das zu untersuchende Fluid und mindestens ein Referenzstrahl zeitversetzt durch einen, das Fluid verdrängenden Kolben oder Kolbenschieber geführt. Dabei kann eine Sammeloptik verwendet werden, bestehend aus mindestens einer Linse, die die Strahlen auf den Auftreffpunkt eines Lichtleiters oder den Einlass des Fotodetektors oder des Spektrometers lenkt, wobei der das Fluid verdrängende Kolben als Strahlenblende dienen kann, die einen Teil der Lichtstrahlen durchlässt und den Rest abschirmt.

Vorteilhaft ist vorgesehen, dass bei Deckungsgleichheit der Unterkante des Kolbens mit der Oberkante der optischen Linsen die spektrometrische Messung durchgeführt wird und die Ergebnisse des Referenzstrahls und die des Messstrahls für jede Wellenlänge oder für Einzelbereiche des Spektrums nach der mathematischen Beziehung

$$E1 = L1 * E_{spez} + C$$

$$E2 = L2 * E_{spez} + C$$

berechnet wird. Es kann vorgesehen sein, dass das Spektrometer die Form einer Sonde hat, dass im Bodenbereich der Sonde der Einlass des Messzylinders in das zu untersuchende Medium eintaucht, dass ein Kolben im Messzylinder entleert und dabei mit seinen Dichtungs- und Reinigungslippen die Innenwandung des Messzylinders reinigt, dass der Kolben zum Befüllen des Messzylinders nach oben fährt, dass bei Deckungsgleichheit der Referenzbohrung mit der optischen Achse die Referenzmessung durchgeführt wird und dass nach Freigabe der optischen Achse die Messung durchgeführt wird.

Der Kolben mit Dichtungs- und Reinigungslippen weist in vorteilhafter Ausgestaltung mindestens eine Durchbohrung in der Zylindermitte und quer zur Zylinderachse für einen Referenzstrahl auf. Diese Durchbohrungsachse liegt parallel zur optischen Achse. Die Messebene wird freigegeben, wenn der Kolben eine Position oberhalb der optischen Linsen einnimmt.

Die Länge des mediumgefüllten Messbereichs, gemessen entlang der optischen Achse, entspricht der Messweglänge des Messstrahls. Abhängig von den zu messenden Substanzen und der notwendigen Messgenauigkeit wird die Messweglänge festgelegt. Bei sehr stark absorbierenden Substanzen wird die Weglänge typischerweise ca. 2-3 mm betragen, bei sehr gering absorbierenden Substanzen 50-100 mm und mehr. Unter technischen Gesichtspunkten gilt eine Weglänge von etwa 5-20 mm als ausreichend für die Messgenauigkeit bei verschiedenen Medien.

Die Kolbendurchbohrung verkleinert den Messstrahl auf die notwendige Flächengröße des Referenzstrahls. Das Flächenverhältnis zwischen Messstrahl und Referenzstrahl beträgt je nach Messmedium 1-5,; daher kann der Kolben erfindungsgemäß weitere übereinander liegende, im Durchmesser abgestufte Referenzbohrungen erhalten, die es dem Rechner ermöglichen, die geeignete Referenzbohrung automatisch auszuwählen.

In Weiterbildung der Erfindung ist daher vorgesehen, dass mindestens ein weiterer, vom Durchmesser des ersten Referenzstrahls verschiedener Referenzstrahl gemessen wird und der Rechner den optimalen Referenzstrahl ermittelt.

Der Messzylinder wird mindestens im Bereich der Linsen vorzugsweise aus UV-beständigem und UV-durchlässigem Quarzglas hergestellt. Vorteilhaft ist es, den Zylinder komplett aus geeignetem Glas zu fertigen; dadurch werden die Übergänge zwischen Glas und anderen Materialien, die zu Störungen führen können, vermieden.

Erfindungsgemäß wird die Aufnahmeeinheit der optischen Ebene, bestehend aus einer Lichtquelle, mindestens einer optischen Linse, die das Licht zu einem im wesentlichen parallelen Strahl bündelt, und mindestens einer optischen Linse, die das Licht nach Verlassen des Messmediums auf den Eintrittspunkt eines Lichtleiters oder den Einlass eines Spektrometers oder Fotodetektors lenkt, aus einem UV-beständigen Montageblock, vorzugsweise aus Rundstabmaterial gefertigt. Dadurch sind die Bauteile in ihrer Achse fixiert. Lampe, Lichtleiter und Linsen können dabei in der Lichtachse zur Feinjustierung verschiebbar eingebaut werden oder fest montiert sein. Die Aufnahmeeinheit der optischen Ebene erhält eine Durchbohrung für den Messzylinder. Dadurch sind die optische Achse und die Messzylinderachse in ihrer Lage fixiert. Die Achsen schneiden sich unter einem Winkel von 90°.

Bei der erfindungsgemäßen Ausgestaltung des Verfahrens zur Messung im Belebungsbecken einer Kläranlage wird der Kolben so langsam nach oben gezogen, dass der Belebtschlamm im unteren Bereich des Messzylinders einen Schwebefilter aus Belebtschlamm aufbaut, so dass sich im Bereich der optischen Achse ein weitgehend feststofffreies Medium ansammelt, und dass der Zustand der weitgehenden Feststofffreiheit durch die optische Messung überprüft wird. Der Kolben kann aber auch mit einer Geschwindigkeit, die größer als die Absetzgeschwindigkeit des Belebtschlammes ist, nach oben gezogen werden. Vorzugsweise wird die Befüllungsgeschwindigkeit zwischen 0,5 und 2,0 cm pro Sek. betragen. In diesem Fall wird dem Messmedium eine Verweilzeit zum Absetzen bis zum Beginn der fotometrischen Messung eingeräumt. Im letztgenannten Befüllungszustand wird bei Erreichen der Kolbenposition oberhalb der optischen Achse in vorgegebenen Sekundenabständen der Trübungsgrad des Mediums gemessen. Nach Erreichen eines vorgegebenen Klärungsgrades leitet der Rechner dann automatisch die fotometrische Messung ein.

Insbesondere kann vorgesehen werden, dass bei der Messung von Belebtschlamm der Kolben zur Befüllung des Messzylinders mit einer solchen Geschwindigkeit nach oben gezogen wird, dass eine Entmischung von Schlamm und Medium nicht stattfinden kann, dass bei Deckungsgleichheit der Unterkante des Kolbens mit der Oberkante der optischen Linsen in vorzugebenden Sekundenabständen der Absetzgrad des Schlamm-Medium-Gemisches optisch vermessen wird und dass bei Erreichen eines weitgehend feststofffreien Mediums in der optischen Achse die eigentliche fotometrische Messung beginnt, und dass die bei der Messung des Absetzgrades gewonnenen Messdaten zur Ermittlung der sog. Schlammtrockensubstanz, des sog. Schlammvolumens und des sog. Schlammindexes ausgewertet werden.

Es kann vorgesehen werden, dass das Licht der Lichtquelle aufgefächert und mittels mindestens einer optischen Linse zu einem im wesentlichen parallelen Strahl gebündelt wird.

Eine Vorrichtung zur Durchführung des Verfahrens mit einer Lichtquelle, einem Fotodetektor, beispielsweise einem Spektrometer, und mindestens einer optischen Linse als Sammeloptik ist dadurch gekennzeichnet, dass die Sonde eine Aufnahmeeinheit für die Lampe , den Lichtleiter und die optischen Linsen enthält und dass die Bauteile dieser optischen Achse in Achsrichtung festmontiert oder verschiebbar angeordnet sind und dass ein Messzylinder, vorzugsweise aus UV-durchlässigem Quarzglas, mit seiner Mittelachse die optische Achse unter einem Winkel von 90° schneidet, wobei der Zylinder senkrecht nach oben weist, dass der Messzylinder mit einem Kolben bestückt ist, wobei der Kolben mit Dichtungs- und Reinigungslippen ausgestattet ist, dass der Kolben mindestens eine Durchbohrung für einen Referenzstrahl enthält und dass diese Durchbohrung in der vertikalen Ebene der optischen Achse verläuft und dass die Achsen parallel zueinander laufen und dass der Kolben durch den Motor mittels einer Antriebsstange bewegt wird, dass die Lampe mit der Versorgungselektronik und der Lichtleiter mit dem Detektor verbunden ist und dass die Versorgungselektronik, der Detektor, der Motor und weitere Bauteile mit der Auswerteeinheit verbunden sind und dass die Auswerteeinheit alle internen Prozesse der Boje steuert und ggf. Daten an einen externen Rechner weiterleitet, dass diese Daten über die Datenleitung weitergegeben werden und dass dieser Leitungsstrang auch zur Energieversorgung der Boje dient.

Nachfolgend werden Ausführungsbeispiele der Erfindung anhand der Zeichnung näher beschrieben. Es zeigt:

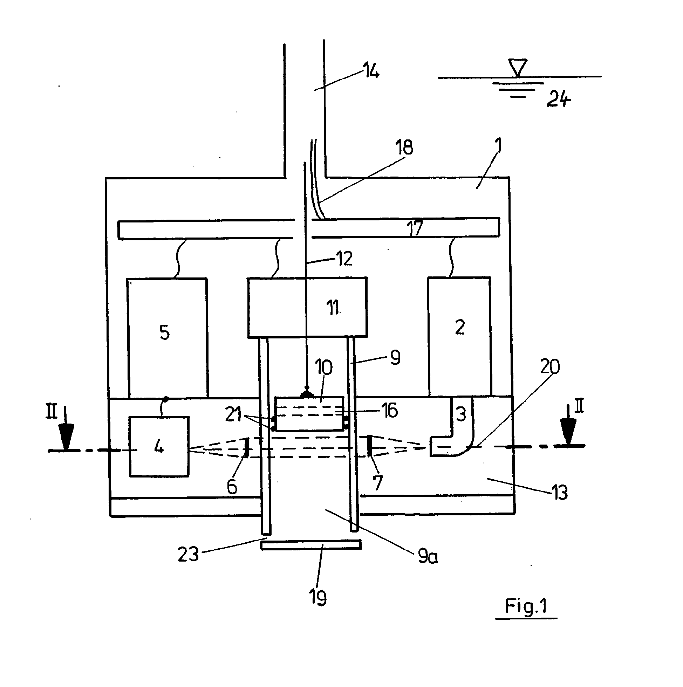

Fig. 1 in vereinfachter Darstellungsweise eine Vorrichtung zur spektrometrischen "in situ"-Messung, wobei die Messsonde im Längsschnitt dargestellt ist,

Fig. 2 einen Schnitt längs der Linie II-II in Fig. 1,

Fig. 3 eine abgewandelte Ausführung einer Fotometrische Sonde und

Fig. 4 einen Schnitt längs der Linie IV-IV in Fig. 3.

Im unteren Teil einer im wesentlichen zylindrischen Sonde 1 ist eine Aufnahmeeinheit 13 für die optischen Bauteile platziert. Eine Lampe 4, ein Lichtleiter 3 und optische Linsen 6 und 7 liegen in ihren optischen Mittelpunkten in der optischen Achse 20. Sie können zur Feinjustierung in Achsenrichtung verschiebbar oder fest montiert sein.

Die optische Wirkung der Linsen 6 und 7 kann auch durch entsprechenden Schliff eines Messzylinders erreicht werden.

Die Lampe 4 ist mit einer Versorgungselektronik 5 und der Lichtleiter 3 ist mit einem optischen Detektor 2 verbunden. Der Messzylinder 9 ist vorzugsweise aus UV-beständigem und UV-durchlässigem Quarzglas hergestellt. Seine Zylinderachse schneidet die optische Achse 20 unter einem Winkel von 90°. Sein Durchmesser liegt zwischen 5 und 30 mm.

Ein Kolben 10 oder Kolbenschieber ist mit Dichtungs- und Reinigungslippen 21 ausgestattet. Eine Referenzbohrung 16 für einen Referenzstrahl verläuft in der vertikalen Ebene der optischen Achse 20 und parallel zu dieser. Der Kolben 10 wird durch einen Motor 11 über eine Antriebsstange 12 bewegt. Ein Bodenstück 19 hat die Aufgabe, eventuell aufsteigende Luftblasen abzuhalten. Öffnungen 23 dienen zur Befüllung und Entleerung des Messzylinders 9.

Eine Auswerteeinheit 17 ist mit dem Detektor 2, dem Motor 11, der Versorgungselektronik 5 und weiteren Bauteilen verbunden und steuert alle internen Prozesse. Sie kann Daten zwischenspeichern und an einen externen Rechner weiterleiten. Eine Leitung 18 dient zur Energieversorgung und Datenübertragung. Ein Befestigungsrohr 14 dient auch zum Herablassen und Hochziehen der Sonde 1.

Ein Messzyklus läuft beispielsweise wie folgt ab: Jeder Messzyklus beginnt mit der Entleerung des Messmediums aus dem Innenraum 9a des Messzylinders 9. Der Kolben 10 fährt nach unten und reinigt dabei mit den Dichtungs- und Reinigungslippen 21 die Innenwandung des Messzylinders 9. Das Medium wird dabei durch die Öffnungen 23 aus dem Messzylinder 9 gedrückt. Zum Befüllen des Messzylinders 9 fährt der Kolben 10, angetrieben durch den Motor 11, nach oben. Bei Deckungsgleichheit der Referenzbohrung 16c mit der optischen Achse 20 blitzt die Lampe 4 ein- oder mehrere Male.

Die Strahlen werden von der Linse.6 so umgeleitet, dass sie das Innere des Messzylinders 9 weitgehend als parallele Strahlen durchdringen. Von der Linse 7 werden diese Strahlen in den Einlass des Lichtleiters 3 umgelenkt. Die Bohrungsachse ist mediumfrei, daher wird bei der Passage der Strahlen durch den aus Quarzglas bestehenden Messzylinder 9 die Extinktion des Glases, einschließlich dessen Trübung und Verschmutzung, gemessen und dient als Referenzstrahl. Überschreitet der Kolben 10 beim Aufwärtsfahren den Strahlenbereich der optischen Achse 20, so blitzt die Lampe 4 erneut und misst die Extinktion C des Glaskolbens (wie oben) und den spezifischen Gesamt-Extinktionsmodul $E_{spez}$ im gesamten Querschnitt des Messkolbens.

Der untere Ausgang des Messzylinders 9 besitzt ein Bodenstück 19, in dem die Ein- und Auslassöffnungen 23 eingearbeitet sind. Der untere Eingang des Messzylinders 9 ist einem Feinsiebfilter ausgestattet.

Der Einfluss eventueller Trübungen, Verfärbungen und Verschmutzungen des Glaszylinders im Bereich des Lichtstrahls kann damit durch den Vergleich von Referenz-und Messstrahl rechnerisch kompensiert werden. Die mathematische Beziehung für jede der gemessenen Lichtwellen lautet:

$$E1 = L1 * E_{spez} + C$$

$$E2 = L2 * E_{spez} + C$$

E1 = gemessene Extinktion im Weg 1
E2 = gemessene Extinktion im Weg 2
L1 = Messweglänge (m)
L2 = Referenzweglänge (m)
$E_{spez}$ = spezifischer Gesamt-Extinktionsmodul des Mediums (1/m)
C = Extinktion des Glaszylinders durch Verfärbung, Trübung und Verschmutzung (-)

Die Lösung erfolgt mit den üblichen mathematischen Methoden.

Bei Wässern mit geringer Verschmutzung kann die Referenzmessung auch im Messraum 9a mit destilliertem Wasser durchgeführt werden.

Bei einer Erweiterung des Verfahrens bei Messungen im Belebungsbecken einer Kläranlage wird der Kolben 10 so langsam nach oben gezogen, dass der Belebtschlamm im unteren Bereich des Messzylinders 9 einen Schwebefilter aus Belebtschlamm aufbaut, so dass sich im Bereich der optischen Achse 20 ein weitgehend feststofffreies Medium sammelt. Überschreitet die Unterkante des Kolbens 10 den oberen Bereich des Strahlenbündels der optischen Achse 20, wird der feststofffreie Zustand des Mediums durch Messung der Absorption überprüft. Bei ausreichender Absetzqualität beginnt die eigentliche fotometrische Messung.

Bei einer weiteren Ergänzung des Verfahrens bei Messungen im Belebungsbecken einer Kläranlage wird der Kolben 10 zur Befüllung des Messzylinders mit einer solchen Geschwindigkeit nach oben gezogen, dass eine Entmischung von Schlamm und Messmedium nicht stattfinden kann, 0,5 und 2,0 cm pro Sek. betragen. Überschreitet die Unterkante des Kolbens 10 den oberen Bereich des Strahlenbündels der optischen Achse 20, so wird der Feststoffgehalt des Belebtschlammes gemessen. In vorzugebenden Sekundenabständen wird danach der Absetzgrad des Medium-Schlamm-Gemisches gemessen, bis ein weitgehend feststofffreies Medium erreicht ist und die eigentliche fotometrische Messung eingeleitet werden kann.

Durch die Messung des Absetzprofils ist das sogenannte Schlammvolumen in ml/l ermittelbar. Zusammen mit dem zuvor ermittelten Feststoffgehalt in g/l steht damit auch ein Ersatzwert für den sogenannten Schlammindex zur Verfügung. Der Schlammindex entspricht dem Schlammvolumen (ml/l) / Feststoffgehalt (g/l).

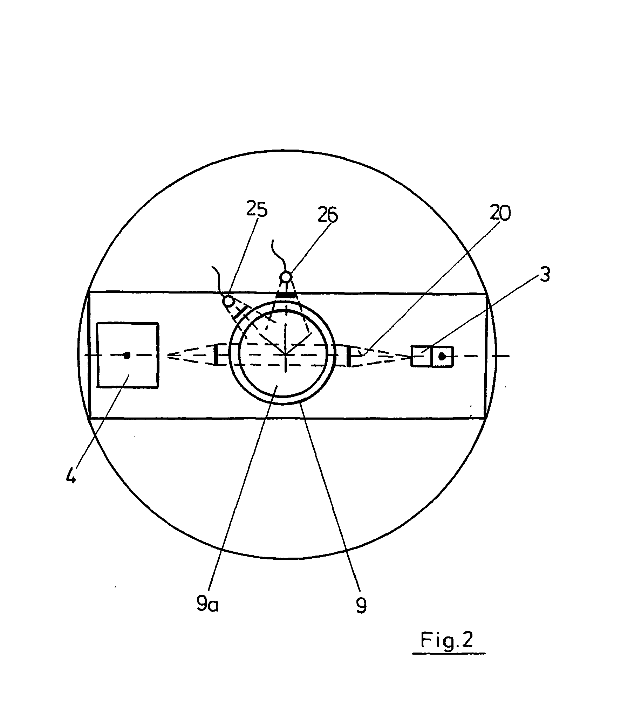

Wie in Fig. 2 gezeigt, können zur Bestimmung der Lichtstreuung seitliche Fotodetektoren 25, 26 vorgesehen sein, die unter vorgegebenen Winkeln von z. B. 45° und/oder 90° zur optischen Achse 20 die Lichtstreuung messen.

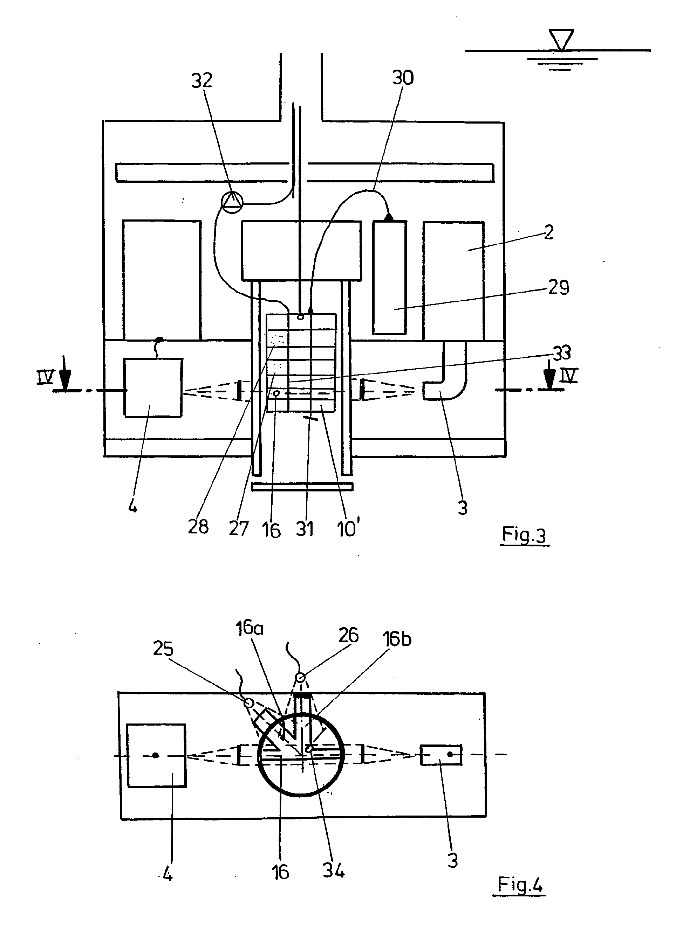

Die Ausführung nach Fig. 3 und 4 unterscheidet sich von der vorher beschriebenen Ausführung im wesentlichen dadurch, dass der Kolben 10' außer der Bohrung 16 mindestens eine weitere Bohrung für den Referenzstrahl, beispielsweise axial versetzt noch weitere Bohrungen 27, 28 aufweist, die zum Zweck der Kalibrierung mit unterschiedlichen Kalibrierflüssigkeiten oder Kalibrierstoffen, beispielsweise Gelen, gefüllt sein können.

Über einen Motor 29 und eine flexible Welle 30 kann ein Rührwerkzeug 31 angetrieben werden, um eine intensive Durchmischung des Fluids zu erreichen. Dies ist besonders zweckmäßig, wenn über eine Reagenzpumpe 32 und eine in den Zylinderinnenraum mündende flexible Reagenzleitung 33 Reagenzien zugegeben werden.

Fig. 4 zeigt, dass von der Referenzbohrung 16, die der Kalibrierung des Fotodetektors 2 dient, Referenzbohrungen 16a und 16b abzweigen, damit auch eine Kalibrierung der Streulichtsensoren 25 und 26 erfolgen kann. Ein Streukörper 34, beispielsweise aus Glas, kann exzentrisch in die Bohrung 16 eingesetzt sein, um eine definierte Streuung zu Kalibrierungsszwecken zu erzeugen.

Der optische Detektor 2 kann ein Spektralfotometer sein, der mit einer zugehörigen Lichtquelle 4 einen Wellenlängenbereich von ca. 190 bis 800 nm für die Spektrometrie abdeckt. Für einfache Anwendungsgebiete kann aber auch der Einsatz einer LED als Lichtquelle und einer einfachen Fotodiode als Empfänger sinnvoll sein. Eine solche Lösung ist sehr einfach umsetzbar und sehr preiswert. Zur Detektion ausgewählter Wellenlängen bieten sich Laserdioden als starke monochromatische Lichtquellen an. In Kombination mit einem geeigneten Fotodetektor lassen sich sehr geringe Konzentrationen nachweisen. Der Einsatz von Infrarotlicht, insbesondere NIR, bietet sich an, wenn Substanzen selektiv in Gemischen zu detektieren sind.

**Patentansprüche**

1. Verfahren zur Bestimmung der Inhaltsstoffe eines flüssigen Mediums mit einer Lichtquelle und einem, Spektrometer, mit mindestens einem Messstrahl und mindestens einem Referenzstrahl, wobei
der Messstrahl durch einen mit dem zu untersuchenden Medium gefüllten Messzylinder geführt wird,
der Referenzstrahl durch den Messzylinder ohne das zu untersuchende Medium geführt wird,
ein längsbeweglicher Kolben das zu untersuchende Medium in einen Messzylinder saugt und aus diesem entfernt, und bei seiner Hubbewegung Fenster im optischen Strahlengang säubert, wobei ferner
das Spektrometer die Form einer Sonde(1) hat,
im Bodenbereich der Sonde(1) der Einlass (23) des Messzylinders (9) in das zu untersuchende Medium (24) eintaucht,
der Kolben (10,10') im Messzylinder nach unten fährt und dabei den Messzylinder (9) entleert und dabei mit seinen Dichtungs- und Reinigungslippen (21) die Innenwandung des Messzylinders (9) reinigt,
der Kolben (10,10') zum Befüllen des Messzylinders (9) nach oben fährt,
bei Deckungsgleichheit einer Referenzbohrung (16) durch den Kolben mit der optischen Achse (20) eine Referenzmessung durchgeführt wird, und
nach Freigabe der optischen Achse (20) nach weiterem Hochfahren des Kolbens eine Messung an dem flüssigen Medium durchgeführt wird.

2. Verfahren nach Anspruch 1, **dadurch gekennzeichnet, dass** bei Deckungsgleichheit der Unterkante des Kolbens (10,10') mit der Oberkante der optischen Linsen (6,7) die spektrometrische Messung durchgeführt wird und die Ergebnisse des Referenzstrahls und die des Messstrahls für jede Wellenlänge oder für Einzelbereiche des Spektrums nach der mathematischen Beziehung

$$E1 = L1 * Espez + C$$

$$E2 = L2 * Espez + C$$

berechnet wird.

3. Verfahren nach Anspruch 1, **dadurch gekennzeichnet, dass** mindestens ein weiterer, vom Durchmesser des ersten Referenzstrahls verschiedener Referenzstrahl gemessen wird und der Rechner den optimalen Referenzstrahl ermittelt.

4. Verfahren nach einem der Ansprüche 1-3, **dadurch gekennzeichnet, dass** bei der Messung von Belebtschlamm der Kolben (10,10') zur Befüllung des Messzylinders (9) so langsam nach oben gezogen wird, dass sich im Bereich der optischen Achse (20) ein weitgehend feststofffreies Medium ansammelt und dass der Zustand der weitgehenden Feststofffreiheit durch die optische Messung überprüft wird.

5. Verfahren nach einem der Ansprüche 1-3, **dadurch gekennzeichnet, dass** bei der Messung von Belebtschlamm der Kolben (10,10') zur Befüllung des Messzylinders (9) mit einer solchen Geschwindigkeit nach oben gezogen wird, dass eine Entmischung von Schlamm und Medium nicht stattfinden kann, dass bei Deckungsgleichheit der Unterkante des Kolbens (10,10') mit der Oberkante der optischen Linsen (6,7) in vorzugebenden Sekundenabständen der Absetzgrad des Schlamm-Medium-Gemisches optisch vermessen wird und dass bei Erreichen eines weitgehend feststofffreien Mediums in der optischen Achse (20) die eigentliche fotometrische Messung beginnt, und dass die bei der Messung des Absetzgrades gewonnenen Messdaten zur Ermittlung der sog. Schlammtrockensubstanz, des sog. Schlammvolumens und des sog. Schlammindexes ausgewertet werden.

6. Verfahren nach einem der Ansprüche 1-5, **dadurch gekennzeichnet, dass** das Licht der Lichtquelle (4) aufgefächert und mittels mindestens einer optischen Linse (6) zu einem im wesentlichen parallelen Strahl gebündelt wird.

7. Vorrichtung zur Durchführung des Verfahrens nach einem der Ansprüche 1-6 mit einer Lichtquelle, einem Fotodetektor, beispielsweise einem Spektrometer, und mindestens einer optischen Linse als Sammeloptik, **dadurch gekennzeichnet, dass** die Sonde(1) eine Aufnahmeeinheit (13) für die Lampe (4), den Lichtleiter (3) und die optischen Linsen (6,7) enthält und dass die Bauteile dieser optischen Achse in Achsrichtung fest montiert oder verschiebbar angeordnet sind und dass ein Messzylinder, vorzugsweise aus UV-durchlässigem Quarzglas, mit seiner Mittelachse die optische Achse (20) unter einem Winkel von 90° schneidet, wobei der Zylinder senkrecht nach oben weist, dass der Messzylinder (9) mit einem Kolben (10,10') bestückt ist, wobei der Kolben mit Dichtungs- und Reinigungslippen (21) ausgestattet ist, dass der Kolben (10) mindestens eine Durchbohrung (16) für einen Referenzstrahl enthält und dass diese Durchbohrung(16) in der vertikalen Ebene der optischen Achse (20) verläuft und dass die Achsen parallel zueinander laufen und dass der Kolben (10,10') durch den Motor (11) mittels einer Antriebsstange (12) bewegt wird, dass die Lampe (4) mit der Versorgungselektronik (5) und der Lichtleiter (3) mit dem Detektor (2) verbunden ist, dass die Versorgungselektronik (5), der Detektor (2), der Motor (11) und weitere Bauteile mit der Auswerteeinheit (17) verbunden sind, dass die Auswerteeinheit (17) alle internen Prozesse der in einer Boje angeordneten Vorrichtung steuert und ggf. Daten an einen externen Rechner weiterleitet, dass diese Daten über die Datenleitung(18) weitergegeben werden und dass dieser Leitungsstrang auch zur Energieversorgung der Boje dient.

**8.** Vorrichtung nach Anspruch 7, **dadurch gekennzeichnet, dass** der untere Ausgang des Messzylinders (9) ein Bodenstück (19) besitzt in dem die Ein- und Auslassöffnungen (23) eingearbeitet sind.

**9.** Vorrichtung nach Anspruch 7, **dadurch gekennzeichnet, dass** der untere Eingang des Messzylinders (9) mit einem Feinsiebfilter ausgestattet ist.

**10.** Vorrichtung nach Anspruch 7, **dadurch gekennzeichnet, dass** der Kolben (10,10') mindestens eine weitere Bohrung (27,28) für einen Referenzstrahl aufweist.

**11.** Verfahren nach Anspruch 7, **dadurch gekennzeichnet, dass** der Durchmesser des Messzylinders (9) zwischen 5 und 30 mm liegt.

**Claims**

**1.** A method of determining the contents of a liquid medium, having a light source and a spectrometer, having at least one measurement beam and at least one reference beam, in which
the measurement beam is guided through a measuring cylinder filled with the medium to be studied,
the reference beam is guided through the measuring cylinder without the medium to be studied,
a piston which is movable in the longitudinal direction draws the medium to be studied into a measuring cylinder and removes it therefrom and, during its stroke movement, wipes clean windows in the optical path of the beam, during which further
the spectrometer is in the form of a probe (1),
in the base region of the probe (1), the inlet (23) of the measuring cylinder (9) reaches into the medium (24) to be studied,
the piston (10, 10') in the measuring cylinder moves downward and in so doing empties the measuring cylinder (9) and at the same time cleans the inside wall of the measuring cylinder (9) by means of its sealing and cleaning lips (21),

the piston (10, 10') moves upward in order to fill the measuring cylinder (9), when the piston has brought a reference bore (16) into alignment with the optical axis (20), a reference measurement is performed, and
once the optical axis (20) has been freed as a result of the piston moving further upward, a measurement is performed on the liquid medium.

**2.** A method according to Claim 1, **characterised in that** when the lower edge of the piston (10, 10') is in alignment with the upper edge of the optical lenses (6, 7) the spectrometer measurement is performed, and the results of the reference beam and those of the measurement beam are calculated for each wavelength or for individual ranges of the spectrum by the mathematical relationship:

$$E1 = L1 * E_{spec} + C$$

$$E2 = L2 * E_{spec} + C.$$

**3.** A method according to Claim 1, **characterised in that** at least one further reference beam of a different diameter from that of the first reference beam is measured and the computer determines the optimum reference beam.

**4.** A method according to one of Claims 1 to 3, **characterised in that** when activated sludge is measured the piston (10, 10') is drawn upward to fill the measuring cylinder (9) slowly enough for a largely solids-free medium to accumulate in the region of the optical axis (20), and **in that** the condition of being largely solids-free is verified by the optical measurement.

**5.** A method according to one of Claims 1 to 3, **characterised in that** when activated sludge is measured the piston (10, 10') is drawn upward to fill the measuring cylinder (9) at a speed such that the sludge and the medium cannot separate out, **in that** when the lower edge of the piston (10, 10') is in alignment with the upper edge of the optical lenses (6, 7) the degree of settling of the mixture of sludge and medium is measured by optical means at intervals of a number of seconds which is to be predetermined, and **in that** when a largely solids-free medium is obtained along the optical axis (20) the actual photometric measurement is begun, and **in that** the measurement data obtained on measuring the degree of settling is evaluated in order to determine the so-called dry substance of sludge, the so-called sludge volume and the so-called sludge index.

6. A method according to one of Claims 1 to 5, **characterised in that** the light from the light source (4) is spread and bundled into a substantially parallel beam by means of at least one optical lens (6).

7. A device for carrying out the method according to one of Claims 1 to 6, having a light source, a photosensor, for example a spectrometer, and at least one optical lens as a collecting optical device, **characterised in that** the probe (1) contains a receiving unit (13) for the lamp (4), the optical waveguide (3) and the optical lenses (6, 7), and **in that** the components of this optical axis are arranged to be mounted in fixed manner or displaceably in the axial direction, and **in that** a measuring cylinder, preferably made of UV-transparent quartz glass, intersects the optical axis (20) with its centre axis at an angle of 90°, with the cylinder pointing perpendicularly upward, **in that** the measuring cylinder (9) is equipped with a piston (10, 10'), the piston being equipped with sealing and cleaning lips (21), **in that** the piston (10) contains at least one through bore (16) for a reference beam, and **in that** this through bore (16) extends in the vertical plane of the optical axis (20), and **in that** the axes run parallel to one another, and **in that** the piston (10, 10') is moved by the motor (11) by means of a drive rod (12), **in that** the lamp (4) is connected to the power supply electronics (5) and the optical waveguide (3) is connected to the detector (2), **in that** the power supply electronics (5), the detector (2), the motor (11) and further components are connected to the evaluation unit (17), **in that** the evaluation unit (17) controls all the internal processes of the device, which is arranged in a buoy, and where appropriate passes data on to an external computer, **in that** this data is passed on by way of the data line (18), and **in that** this connection cable also serves to supply power to the buoy.

8. A device according to Claim 7, **characterised in that** the lower output of the measuring cylinder (9) has a base part (19) in which the inlet and outlet openings (23) are made.

9. A device according to Claim 7, **characterised in that** the lower input of the measuring cylinder (9) is equipped with a fine screen filter.

10. A device according to Claim 7, **characterised in that** the piston (10, 10') has at least one further bore (27, 28) for a reference beam.

11. A device according to Claim 7, **characterised in that** the diameter of the measuring cylinder (9) is between 5 and 30 mm.

**Revendications**

1. Procédé destiné à la détermination des composants d'un fluide liquide à l'aide d'une source lumineuse et d'un spectromètre, avec au moins un faisceau de mesure et au moins un faisceau de référence, procédé pour lequel

   le faisceau de mesure est dirigé à travers un cylindre de mesure rempli du fluide à analyser,

   le faisceau de référence est dirigé à travers le cylindre de mesure sans le fluide à analyser,

   un piston déplaçable dans le sens longitudinal aspire le fluide à analyser dans un cylindre de mesure et le retire de ce dernier et nettoie lors de sa course les fenêtres se trouvant dans la trajectoire du faisceau optique, par ailleurs procédé pour lequel

   le spectromètre a la forme d'une sonde (1),

   l'entrée (23) du cylindre de mesure (9) s'immerge dans le fluide à analyser (24) dans la partie inférieure de la sonde (1),

   le piston (10, 10') se déplace vers le bas dans le cylindre de mesure et vide en même temps le cylindre de mesure (9) et nettoie en même temps avec ses lèvres d'étanchéité et de nettoyage (21) la paroi intérieure du cylindre de mesure (9),

   le piston (10, 10') se déplace vers le haut pour le remplissage du cylindre de mesure (9),

   une mesure de référence est effectuée en cas de convergence d'un alésage de référence (16) traversant le piston avec l'axe optique (20) et

   une mesure du fluide est réalisée après validation de l'axe optique (20) après une remontée du piston.

2. Procédé selon la revendication 1, **caractérisé en ce que** la mesure spectrométrique est réalisée en cas de convergence du bord inférieur du piston (10, 10') avec le bord supérieur des lentilles optiques (6, 7) et **en ce que** les résultats du faisceau de référence et du faisceau de mesure sont calculés pour chaque longueur d'onde ou pour des zones individuelles du spectre d'après la relation mathématique

$$E1 = L1 \times E_{spéc.} + C$$

$$E2 = L2 \times E_{spéc.} + C$$

3. Procédé selon la revendication 1, **caractérisé en ce qu'**au moins un faisceau de référence supplémentaire, différent du diamètre du premier faisceau de référence, est mesuré et que l'ordinateur détermine le faisceau de référence optimal.

4. Procédé selon les revendications 1 à 3, **caractérisé**

**en ce que**, lors de la mesure de boue activée, le piston (10, 10') est tiré lentement vers le haut en vue du remplissage du cylindre de mesure (9), de telle sorte que s'accumule dans la zone de l'axe optique (20) un fluide largement exempt de matières solides et que l'état de quasi absence de matières solides est contrôlé par la mesure optique.

**5.** Procédé selon les revendications 1 à 3, **caractérisé en ce que**, lors de la mesure de boue activée, le piston (10, 10') est tiré lentement vers le haut en vue du remplissage du cylindre de mesure (9), à une vitesse telle qu'une dissociation de boue et de fluide ne puisse avoir lieu, **en ce que** le degré de décantation du mélange boue-fluide est mesuré de façon optique, selon des intervalles de temps en secondes à prédéfinir, en cas de convergence du bord inférieur du piston (10, 10') avec le bord supérieur des lentilles optiques (6, 7), et que la mesure photométrique proprement dite commence dans l'axe optique (20) dès l'obtention d'un fluide largement exempt de matières solides, et que les données de mesure acquises lors de la mesure du degré de décantation sont exploitées en vue de la détermination de la dite substance sèche de la boue, le dit volume de boue et le dit indice de boue.

**6.** Procédé selon les revendications 1 à 5, **caractérisé en ce que** la lumière de la source lumineuse (4) est dispersée et focalisée au moyen d'un lentille optique (6) en un faisceau essentiellement parallèle.

**7.** Dispositif destiné à la réalisation du procédé d'après l'une des revendications 1 à 6 avec une source lumineuse, un photodétecteur, par exemple un spectromètre, et au moins une lentille en tant que système optique global, **caractérisé en ce que** la sonde (1) contient une unité-support (13) pour la lampe (4), le guide d'onde (3) et les lentilles optiques (6, 7), et que les composants de cet axe optique sont montés dans la direction de l'axe, soit de façon fixe, soit de façon mobile, et qu'un cylindre de mesure, de préférence en verre de quartz transparent aux UV, coupe avec son axe médian l'axe optique (20) selon un angle de 90°, le cylindre étant orienté verticalement vers le haut, **en ce que** le cylindre de mesure (9) est muni d'un piston (10, 10'), le piston étant doté de lèvres d'étanchéité et de nettoyage (21), **en ce que** le piston (10) contient au moins un alésage traversant (16) pour un faisceau de référence et que cet alésage traversant (16) se trouve dans le plan vertical de l'axe optique (20) et que les axes sont parallèles l'un par rapport à l'autre et que le piston (10, 10') est déplacé par le moteur (11) au moyen d'une tige d'entraînement (12), **en ce que** la lampe (4) est reliée avec le système électronique d'alimentation (5) et le guide d'onde (3) avec le détecteur (2), **en ce que** le système électronique d'alimentation (5), le détecteur (2), le moteur (11) et d'autres composants sont reliés avec l'unité d'exploitation (17), **en ce que** l'unité d'exploitation (17) pilote tous les processus internes dans un dispositif disposé dans une bouée et, le cas échéant, transmet les données à un ordinateur externe, **en ce que** les données sont transmises par l'intermédiaire de la ligne de données (18) et que ce faisceau de câbles sert également d'alimentation en énergie de la bouée.

**8.** Dispositif selon la revendication 7, **caractérisé en ce que** la sortie inférieure du cylindre de mesure (9) est munie d'une pièce de fond (19), dans laquelle ont été réalisées des ouvertures d'entrée et de sortie (23).

**9.** Dispositif selon la revendication 7, **caractérisé en ce que** l'entrée inférieure du cylindre de mesure (9) est munie d'un filtre à tamis fin.

**10.** Dispositif selon la revendication 7, **caractérisé en ce que** le piston (10, 10') comporte au moins un alésage (27, 28) supplémentaire pour un faisceau de référence.

**11.** Dispositif selon la revendication 7, **caractérisé en ce que** le diamètre du cylindre de mesure (9) est compris entre 5 et 30 mm.

Fig.1

Fig.2

Fig.3

Fig.4